# EUROPÄISCHE PATENTSCHRIFT

(11) **EP 1 174 145 B1**
(45) Veröffentlichungstag und Bekanntmachung des Hinweises auf die Patenterteilung: **25.01.2006**
(21) Anmeldenummer: 01810689.8
(22) Anmeldetag: 11.07.2001
(51) Int. Cl.: A61K 36/00, B01D 11/02

(54) **Extraktion von Wirkstoffen aus Pflanzen**
Extraction of active agents from plants
Extraction d'agents actifs de plantes

(30) Priorität: 21.07.2000 CH 20001440
(43) Veröffentlichungstag der Anmeldung: 23.01.2002
(73) Patentinhaber: Fondation The Ark, 1950 Sion (CH)
(72) Erfinder: Grogg, Alain-François, 1965 Savièse (CH); Antille, Laurence, 3960 Sierre (CH); Zonnevijlle, Frans, 1762 Givisiez (CH); Tobler, Martin, 9303 Wittenbach (CH); Furrer, Roland, 9000 St. Gallen (CH)
(74) Vertreter: Breiter, Heinz

(56) Entgegenhaltungen:
- GB-A- 410 301
- US-A- 5 800 817
- DATABASE BIOSIS [Online] BIOSCIENCES INFORMATION SERVICE, PHILADELPHIA, PA, US; 1985 RONDINA R V D ET AL: "STANDARD METHOD FOR THE SYSTEMATIC PREPARATION AND FRACTIONATION OF PLANT EXTRACTS TO TRACE THEIR PHARMACOLOGICAL ACTIVITY AND FOR CHEMICAL STUDY" Database accession no. PREV198681046341 XP002212492 & ACTA FARMACEUTICA BONAERENSE, Bd. 4, Nr. 1, 1985, Seiten 3-14, ISSN: 0326-2383

## Beschreibung

Die Erfindung bezieht sich auf ein Verfahren zur Fest-Flüssig-Extraktion von Wirkstoffen aus fettarmen Pflanzen oder Pflanzenteilen mit einem Extraktionslösungsmittel zur Herstellung von Pflanzenextrakten. Weiter betrifft die Erfindung Verwendungen der mit dem Verfahren gewonnenen Wirkstoffe.

Nach bekannten Verfahren wird bei der Herstellung von Extrakten aus fettarmen Pflanzen oder Pflanzenteilen meist zuerst das Pflanzenmaterial zerkleinert, damit die Extraktionskontaktfläche, auch Grenzfläche genannt, und somit die Extraktionseffizienz vergrössert wird. Diese Extraktionsverfahren werden mit mittels Trocknung wenigstens teilweise entwässertem Pflanzenmaterial durchgeführt. Aus fettarmen Pflanzen werden keine Oele oder Fette zum Verzehr oder zu Schmierzwecken gewonnen.

Nach der Entwässerung und einem einfachen Zerkleinerungsvorgang, oder auch umgekehrt, können verschiedene Fest-Flüssig-Extraktionsverfahren problemlos durchgeführt werden. Die Gründe für den Einsatz von getrockneten Pflanzenmaterial sind in erster Linie von mikrobiologischer und/oder logistischer Natur, sie hängen jedoch auch mit der gewählten Extraktionstechnik zusammen. Eine vorangehende Entwässerung beinhaltet aber auch Nachteile, beim Trocknungsvorgang wird die in der Pflanze vorhandene Wasserphase, die als disperse Phase eines heterogenen Systems angesehen werden kann, teilweise entfemt. Dies führt beispielsweise zu chemischen Veränderungen ihrer Bestandteile, zu einem Verlust an flüchtigen Wirkstoffen und/oder zu einer unerwünschten Zunahme der Sprödigkeit der Zellwände. In allen bekannten Fällen nimmt jedoch die Qualität der Extrakte ab.

Beim Einsatz von fettarmem Pflanzenmaterial führen die bekannten Zerkleinerungsverfahren einerseits zu einem Brei, mit dem eine Fest-Flüssig-Extraktion nur schwer durchzuführen ist, während andererseits diese Extraktion schnell durchgeführt werden muss, um die enzymatische Zersetzung der Wirkstoffe zu verhindern.

Im Detail laufen übliche Verfahren zur Fest-Flüssig-Extraktion von Wirkstoffen aus fettarmen Pflanzen oder Pflanzenteilen mit einem Extraktionslösungsmittel zur Herstellung vom Pflanzenextrakten wie folgt ab. Durch Trocknen werden die frisch geernteten Pflanzen bis zu einem gewissen Grad stabilisiert und können besser gelagert werden. Der nächste übliche Verfahrensschritt ist das Zerkleinern der Pflanzen, wodurch wie erwähnt ihre Extrahierbarkeit erhöht wird. Je feiner die Zerstückelung, desto grösser ist grundsätzlich die Extraktionseffizienz. Zur abschliessenden Extraktion werden beispielsweise verschiedene bekannte Verfahren angewendet:
- Eintauchen der Pflanzen oder Pflanzenteile in ein ruhendes Lösungsmittel bei Zimmertemperatur (Mazeration).
- Mazeration bei erhöhter Raumtemperatur von etwa 50°C (Digestion).
- Das Lösungsmittel fliesst über ein Bett von Pflanzen oder Pflanzenteilen, bei Raumtemperatur oder einer erhöhten Temperatur (Perkolation).

In der DE, A1 2641994 wird ein Verfahren und eine Vorrichtung für die kontinuierliche oder diskontinuierliche Extraktion von löslichen Substanzen aus Feststoffteilchen beschrieben, insbesondere von Ölen und Fetten aus pflanzlichem Material. Dabei wird das flüssige Extrakt durch Senken des Siedepunktes mittels Druckabsenkung bewirkt. Darauf wird der Druck wieder erhöht und mit neuem Lösungsmittel die Extraktion fortgesetzt. Diese Druckänderungen bzw. Lösungsmittelzugaben können mehrmals wiederholt werden.

In der EP, B1 0312855 wird ein Verfahren und eine Vorrichtung zur Gewinnung von pflanzlichen Fetten und Ölen aus ölhaltigen Naturstoffen beschrieben. Zuerst werden diese Naturstoffe mit einem Lösungsmittel gemischt, dann werden sie zerkleinert und das Ganze wieder gemischt. Die Extraktion erfolgt auf einem Temperaturniveau zwischen Raumtemperatur und wenige Grade unter dem Siedepunkt des Lösungsmittels, vorzugsweise in einer Rotor-Stator-Maschine. Abschliessend werden die Lösungsmittel aus den Ölen bzw. Fetten abdestilliert.

Schon aus der NL,C 43417 ist ein kontinuierliches Extraktionsverfahren von öl- und fetthaltigem, pflanzlichem Material bekannt, insbesondere von Ölsamen und Ölfrüchten, mit Lösungsmitteln im Gegenstrom. Die Samen werden feingemahlen und mehrmals zwischen Walzen durchgepresst. Im konkreten Ausführungsbeispiel werden Erdnüsse erwähnt, wo das Verfahren offensichtlich der Extraktion von Erdnussöl dient. Das Lösungsmittel Benzin wird abschliessend entfernt. Frisches Pflanzenmaterial ist zur Durchführung dieses Verfahrens nicht geeignet.

Alle Schriften beziehen sich auf die Extraktion von Ölen und Fetten ohne einen Hinweis auf Wirkstoffe oder für deren Verwendung. Öle und Fette zum Verzehr oder zu Schmierzwecken sind keine Extrakte im erfindungsgemässen Sinn.

Der Erfindung liegt die Aufgabe zugrunde, ein Verfahren der eingangs genannten Art zu schaffen, das unter Vermeidung von Wirkstoffverlusten eine hohe Qualität von extrahierten Pflanzenextrakten gewährleistet. Weiter sollen mit geringerem Vorrichtungsaufwand grössere Pflanzenextraktmengen gewonnen und deren Verwendung aufgezeigt werden.

Die Aufgabe wird bezüglich des Verfahrens erfindungsgemäss dadurch gelöst, dass frischgeschnittene, fettarme Pflanzen oder Pflanzenteile unter Beibehaltung von deren Grundstruktur zu einem heterogenen Flüssig-Fest-Suspensionssystem mit feinstückigem Pflanzenmaterial zerkleinert und die Wirkstoffe im gleichen Arbeitsgang kontinuierlich oder sequentiell mit wenigstens einem Lösungsmittel extrahiert und angereichert werden. Spezielle und weiterführende Ausführungsformen des Verfahrens sind Gegenstand von abhängigen Patentansprüchen.

Erfindungsgemäss wird darauf geachtet, dass die Extraktion des frischen Pflanzenmaterials schnell, schonend und thermisch kontrolliert abläuft, so kann eine hohe Qualität des Pflanzenextraktes gewährleistet und insbesondere die enzymatische Zersetzung von Wirkstoffen verhindert werden. Vorzugsweise wird schon vor dem und/oder beim Zerkleinerungs- bzw. Schneidevorgang etwas Lösungsmittel zugegeben, was als "unter Niveau" bezeichnet wird. Auch Frischpflanzen aus biologischem Anbau werden mit diesem neuen Extraktionsverfahren direkt, ohne vorangehende Trocknung, verarbeitet. Das Zerkleinern erfolgt vorzugsweise durch Schneiden.

Die Extraktion von Ölen und Fetten aus Pflanzensamen mit hohem Öl-und/oder Fettanteil ist ausdrücklich nicht Gegenstand der vorliegenden Erfindung.

Die Erntezeit der Pflanzen oder Pflanzenteile bedingt in der Praxis einen Erntekalender, damit die Pflanzen optimal genutzt werden können. Ein Emtekalender verschiedener Pflanzen sieht beispielsweise wie folgt aus:
- Hypericum herba Juni - August
- Echinacea herba August - September
- Aesculus fructus Oktober
- Echinacea radix Oktober - November
- Valeriana radix November oder März

Mit der Verarbeitung jeder Pflanze ist ein spezifisches Analytikprogramm verbunden, mit dem die Qualität der ausgeführten Extraktionen beurteilt werden kann. In den vorstehend aufgeführten Beispielen werden folgende Wirkstoffe bestimmt (Nativextraktgehalt):
- Hypericum herba: Flavonoide, Hypericine, Hyperforine und Chlorogensäurederivate.
- Echinacea herba: Alkylamide, Cichoriensäure, weitere Kaffeesäurederivate und Rutosid.
- Aesculus fructus: Triterpenglykoside (Aescin).
- Echinacea radix: Alkylamide, Cichoriensäure, weitere Kaffeesäurederivate und Rutosid.
- Valeriana radix: Iridoïde, Kaffeesäurederivate.

Die Extraktion der pflanzlichen Wirkstoffe soll wie erwähnt schnellstmöglich ausgeführt werden, mit andern Worten soll die Extraktionszeit des Pflanzenmaterials im Extraktor durch entsprechende Zerkleinerung möglichst kurz sein. Eine schnelle und kräftige Zerstückelung kann beispielsweise mit einem Turboschneider erfolgen. Die Zerkleinerung erfolgt zweckmässig durch Schneiden bis zum Erreichen einer möglichst homogenen Stückgrösse von 0,1 - 20 mm, bevorzugt 0,5 - 5 mm. Das Verfahren soll für die verschiedensten Arten von Pflanzen und für die wichtigsten Wirkstoffkategorien anwendbar sein, die möglichst homogene Stückgrösse ist pflanzenspezifisch, z.B. etwa 3 mm. Das Verfahren soll in Bezug auf die Art, in der das Extraktionslösungsmittel eingespeist wird (beispielsweise Gegen- und/oder Kreuzstromtyp), ebenfalls flexibel sein. Dieses Lösungsmittel ist, wie das Wasser aus den Pflanzen, Bestandteil des Endproduktes.

Die Schlüsselparameter des Verfahrens sind
- Inhibierung der enzymatischen Aktivitäten durch Eintauchen des frischen Pflanzenmaterials in das Extraktionslösungsmittel (unter Niveau),
- die mittlere Extraktionszeit, d.h. die Kontaktzeit des Lösungsmittels mit der Grenzfläche des feinteiligen Pflanzenmaterials im Extraktor, verhindert weitgehend eine enzymatische Zersetzung der Inhaltstoffe, vorzugsweise im Bereich von 10 - 60 Minuten, insbesondere von 20 - 30 Minuten,
- die Homogenität der Grenzflächen, also die Qualität der Zerkleinerung der frischen Pflanzen oder Pflanzenteile,
- die Leistung des Systems,
- die Temperatur, welche mit Sensoren überwacht wird, und
- Schubspannungen, insbesondere im Zusammenhang mit Förderschnecken.

Die Optimierung der Prozessbedingungen für die Extraktion erfolgt anhand von standardmässigen quantitativen oder halbquantitativen analytischen Techniken, insbesondere mittels Dünnschichtchromatografie-Analysen.

Als Extraktionslösungsmittel, kurz gesagt Lösungsmittel, sind alle anorganischen und organischen Flüssigkeiten geeignet, welche die Wirkstoffe der feinstückig zerkleinerten Pflanzen in kurzer Zeit vollständig oder nahezu vollständig aufzulösen vermögen. Besonders geeignet sind Alkohol und/oder Wasser, insbesondere eine Mischung aus Ethanol/Wasser ergibt sehr gute Qualitätswerte. Weitere Beispiele von eingesetzten Lösungsmitteln sind Glycerin oder Hexan, auch Emulsionen Hexan/Wasser. Zusätzliche Verbesserungen der Qualität des Pflanzenextraktes ergeben sich, wenn das Verfahren in inerter Gasphase durchgeführt wird, beispielsweise durch Zuleitung von Argon oder Stickstoff, wodurch die enzymatische Zersetzung vermindert wird.

Wird die Extraktionszeit variiert, kann die enzymatische Zersetzung untersucht und die Wirksamkeit der Extraktion analysiert werden. Weiter können, ausgehend von physikalisch-chemischen Modellen an sich bekannter Art, der Wirkungsgrad und die Selektivität der Extraktion analytisch im Labor untersucht und die operativen Bedingungen des industriellen Trennverfahrens anhand dieser Resultate optimiert werden.

Eine zweckmässig durchgeführte abschliessende Pressung des Rückstands wird unmittelbar anschliessend an die Extraktion ausgeführt, z.B. mit Hilfe einer Endlosschnecke. Die Arbeitsbedingungen dazu, insbesondere der am Schluss ausgeübte Druck, entsprechen dem üblichen Stand der Technik.

Die Qualität der Pflanzenextrakte kann systematisch, je nach Extraktionszeit, mit der Qualität von Vergleichsproben verglichen werden. Die Verhinderung einer enzymatischen Zersetzung der Schlüsselwirkstoffe ist bei den ausgetesteten Verfahren gesamthaft berücksichtigt worden. Weil die enzymatische Zersetzung eng mit den Einheitsoperationen Zerschneiden und/oder Auspressen verbunden ist, werden einfache Änderungen durchgeführt und der Durchfluss bei den entsprechenden Operationen variiert, damit die unerwünschte Enzymaktivität vermindert oder unterbunden werden kann. Schliesslich kann das gewonnene flüssige Pflanzenextrakt je nach Verwendung allenfalls durch Abdestillieren oder Zugabe von Lösungsmittel beliebig aufkonzentriert werden.

Die Vorteile des erfindungsgemässen Verfahrens können wie folgt zusammengefasst werden:
- Pflanzliche Extrakte können ausgehend von Frischpflanzen oder Frischpflanzenteilen durch effiziente Fest-Flüssig-Extraktion von deren Wirkstoffen hergestellt werden.
- Auf dieser Basis ist es möglich, neue Hochleistungsanlagen zu bauen, welche für die Herstellung von hochaktiven Extrakten optimal sind, eine Sicherung der Qualität der industriell hergestellten Folgeprodukte erlauben und die Wirkstoffverluste durch Verdunstung, chemische oder enzymatische Umsetzung auf einem Minimum halten.

Das mit dem erfindungsgemässen Verfahren gewonnene flüssige Pflanzenextrakt wird insbesondere zur Herstellung von wenigstens teilweise natürlichen Arzneimitteln, Lebensmitteln oder Kosmetika als Folgeprodukte verwendet. Dazu werden an sich bekannte Herstellungsverfahren angewendet, welche nicht mehr Gegenstand der vorliegenden Erfindung sind.

Natürliche Arzneimittel beispielsweise werden immer mehr, allein oder in Kombination mit synthetischen Arzneimitteln, verwendet bzw. vom Patienten verlangt. Dies liegt im aktuellen Trend zur Nachfrage nach natürlichen Produkten. Grundstoff für pflanzliche Arzneimittel sind die qualitativ hochstehende Pflanzenextrakte, welche in verschiedenen galenischen Formen eingesetzt werden können.

Die Erfindung wird anhand von in der Zeichnung dargestellten Ausführungsbeispielen, welche auch Gegenstand von abhängigen Patentansprüchen sind, näher erläutert. Es zeigen schematisch:
- Fig. 1 eine Fliessbilddarstellung einer Rührkessel-Extraktoranlage,
- Fig. 2 ein Fliessbild einer Schnecken-Extraktoranlage, und
- Fig. 3 ein Fliessbild einer Rührkessel-Kaskadenanlage.

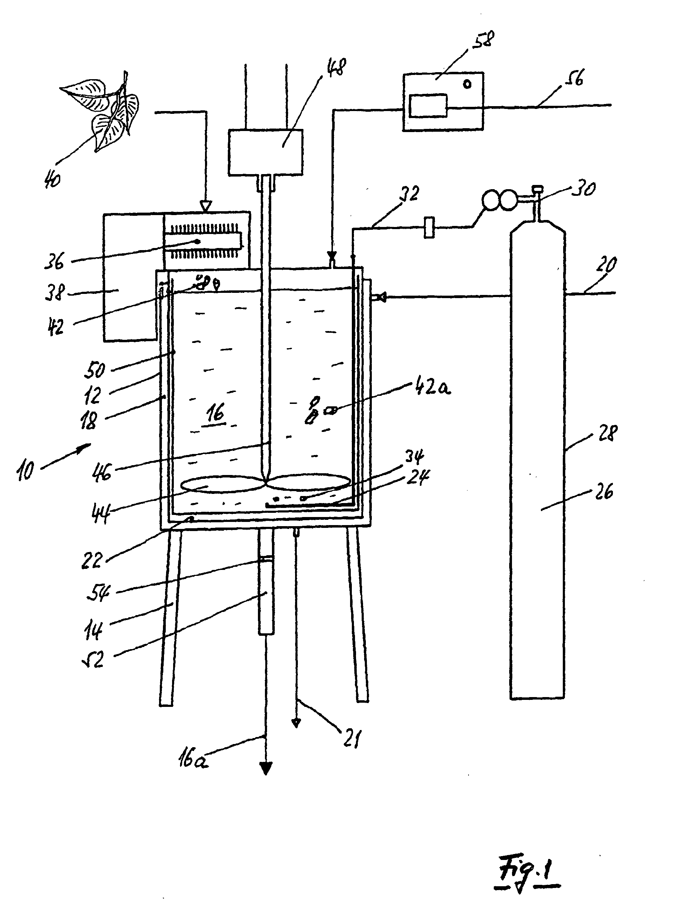

Fig. 1 zeigt eine Rührkessel-Extraktionsanlage 10 mit einem auf vier Beinen 14 gelagerten Kessel 12. Dieser Kessel ist mit einem Extraktionslösungsmittel 16 gefüllt, im vorliegenden Fall eine Mischung aus Ethanol/Wasser.

Der Kessel 12 weist einen Kühlmantel 18 auf, welcher mit einer Kühlmittelzufuhrleitung 20 gespeist wird. Die Abflussleitung 21 des Kühlmittels ist ebenfalls angedeutet.

Knapp oberhalb des Bodens 22 des Rührkessels 12 ist eine Düsenleiste 24, sich mindestens über einen Teil dieses Bodens erstreckend, angeordnet, aus welcher im Betrieb Inertgasblasen 34 ausströmen, insbesondere Argon.

Eine mit Inertgas 26 gefüllte Druckflasche 28 gibt über ein Reduzierventil 30 und eine Druckleitung 32 Argon ab, welches über die Düsenleiste 24 eingespeist wird.

Unmittelbar oberhalb des Rührkessels 12 ist ein von einem Motor 38 angetriebener Zerkleinerer 36 montiert, welcher einen nicht dargestellten Einzugstrichter haben kann. Dem Zerkleinerer 36, beispielsweise ein Turboschneider oder ein mit Messern versehener Walzenkneter, werden frische Pflanzen oder Pflanzenteile 40 zugeführt und zerkleinert. Das feinstückige Pflanzenmaterial 42 fällt in das Extraktionslösungsmittel 16, wo es von einem Rührer 44 aufgewirbelt wird. Es entsteht ein feinstückiges heterogenes Flüssig-Fest-Suspensionssystem, welches im wesentlichen aus dem Extraktionslösungsmittel 16 mit teilweise extrahierten Wirkstoffen für pflanzliche Arzneimittel, dem Extrakt 16a, aufsteigenden Inertgasblasen 34 und feinstückigem Pflanzenmaterial 42a besteht.

Der Rührer 44 ist über eine Welle 46 angetrieben, welche ihrerseits von einem am nicht dargestellten Maschinengehäuse oder -rahmen gehalterten Motor 48 rotiert wird.

Ein den Rührkessel 12 auskleidender, gas- und flüssigkeitsdurchlässiger Metallkorb 50 mit einem nicht erkennbaren Filtertuch kann zum Entfernen des vollständig extrahierten feinstückigen Pflanzenmaterials 42a abgehoben und entleert werden, natürlich erst nach dem Entfernen der behindernden Anlageteile. Das abfiltrierte Lösungsmittel 16 mit den Pflanzen entnommenen Wirkstoffen wird durch einen mittels eines Ventils 54 verschliessbaren Stutzen 52 abgelassen und als Extrakt 16a der weiteren Verarbeitung zugeführt. Selbstverständlich muss das Extrakt nicht nach jedem Entfernen einer Charge von feinstückigem Pflanzenmaterial 42a abgelassen werden.

Eine Zufuhrleitung 56 für das Lösungsmittel 16 führt über eine Dosiervorrichtung 58 in den Rührkessel 12.

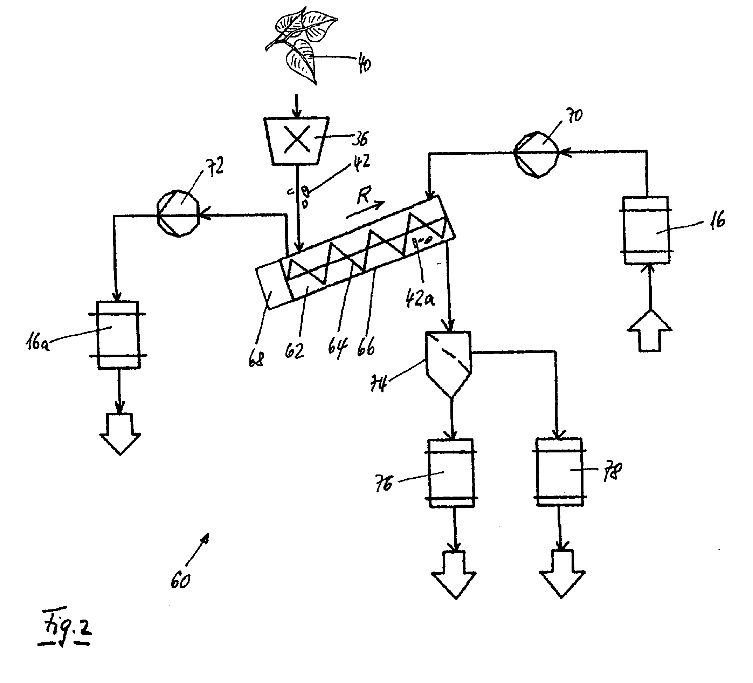

In Fig. 2 ist eine Schnecken-Extraktoranlage 60 dargestellt. Ein Schnecken-extraktor 62 arbeitet im vorliegenden Fall nach dem bekannten Hildebrandt-Prinzip. Für die vorliegend dargestellte Laborextraktionsanlage besteht er aus einer in einem Rohr 66 von 55 mm Innendurchmesser drehenden Schnecke 64 von 55 mm Aussendurchmesser. Die Schnecke 64 ist aus einem Band oder Draht von 6 mm Durchmesser gebogen worden. Die Länge der Schnecke 64 beträgt 850 mm, sie kann jedoch mittels eines Verlängerungsteils auf 1400 mm erhöht werden. Die einzige Fixierung der Schnecke 64 mit einer Steigung von 30 mm ist die Verbindung zu einem Antriebsmotor 68, welcher drehzahlvariabel ist und bei einer maximalen Drehzahl von etwa 1380 pro Minute eine Leistung von etwa 250 Watt erbringen kann. Das Rohr 66 ist gegenüber der Horizontalen mit einem Winkel von etwa 20° geneigt. Die normale Drehgeschwindigkeit der Walze 64 liegt bei etwa 0,75 Umdrehungen pro Minute.

Eine Zahnradpumpe 70 lässt das Lösungsmittel 16 im Gegenstrom zur Förderrichtung R des feinstückigen Pflanzenmaterials 42 das Rohr 66 abwärts fliessen. Die frischen Pflanzen oder Pflanzenteile 40 werden zuerst unter Niveau zerkleinert, d.h. alles eingespeiste Lösungsmittel 16 kann zum Aufbau eines feinstückigen heterogenen Flüssig-Fest-Suspensionssystems aufgenommen werden. Im unteren Bereich der Schnecke 64 wird das nunmehr mit Wirkstoffen angereicherte Lösungsmittel 16 aufwärts transportiert. Das Gegenstromprinzip hat den Vorteil, dass die Konzentrationsunterschiede an Wirkstoffen von Extrakt und Frischpflanze immer möglichst gross gehalten werden können. Wie bereits erwähnt, hängt das Ausmass der Extraktion massgeblich von der Extraktionszeit ab, welche hauptsächlich durch die Schneckenform und -länge, den Füllungsgrad und die Drehzahl der Schnecke 64 bestimmt wird. Die besten Resultate werden mit Doppelschnecken erzielt.

Eine Pumpe 72 entzieht, zweckmässig sensorgesteuert, im unteren Bereich des Schnecken-Extraktors 62 filtriertes Extrakt 16a, d.h. mit Wirkstoffen angereichertes Lösungsmittel 16.

Das kontinuierlich aus dem Schnecken-Extraktor 62 ausgestossene feinstückige Pflanzenmaterial 42a, welchem die Wirkstoffe weitgehend oder vollständig entzogen sind, fällt in einen Separator 74, welcher das feuchte feinstückige Pflanzenmaterial 42a ohne die entzogenen Wirkstoffe in ein Raffinat 76 und einen Trester 78 aufteilt. Der Trester 78 wird zweckmässig ausgepresst.

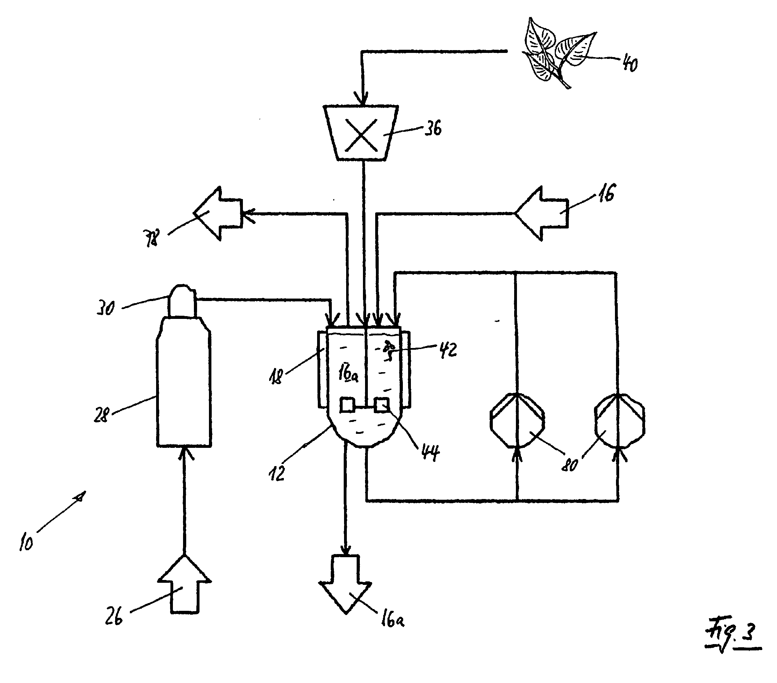

In Fig. 3, einer Weiterentwicklung von Fig. 1, ist der Rührkessel 12 als Rührkesselkaskade für die sequentielle Extraktion ausgebildet. Genauer gesagt wird das Verhalten einer üblichen Rührkesselkaskade mit wesentlich grösserem Konstruktionsaufwand simuliert. Dem Rührkessel 12 wird im untersten Bereich Extrakt 16a, also mit Wirkstoffen angereichertes Extraktionslösungsmittel 16, entzogen und mit peristaltischen Pumpen 80 wieder in den Rührkessel 12 gefördert. Durch diese im wesentlichen aus Lösungsmittel 16 bestehende Flüssigkeitszirkulation soll eine Durchmischung im Rührkessel 12 gewährleistet werden. Bei einer vorgegebenen Anreicherung mit Wirkstoffen wird das Extrakt 16a aus Wirkstoffen und Lösungsmittel 16 abgezogen und durch eine entsprechende Zugabe von frischem Lösungsmittel 16 kompensiert.

Im übrigen entspricht die Rührkessel-Extraktionsanlage 10 im wesentlichen derjenigen von Fig. 1. Der Rührkessel 12 ist innen 290 mm hoch und hat einen lichten Durchmesser von 340 mm. Der Übersichtlichkeit wegen ist der Zerkleinerer 36 in Abstand oberhalb des Rührkessels 12 eingezeichnet, in Wirklichkeit ist er unmittelbar darauf angeordnet. Der Metallkorb 50 mit Filtriertuch ist weggelassen. Der zur kontinuierlichen Durchmischung von feinstückigem Pflanzenmaterial 42 und Lösungsmitteln 16 dienende Rührer 44 ist als Scheibenrührer ausgebildet.

Die Begasung mit einem Inertgas 26 von unten, insbesondere Argon, ist wesentlich, weil dadurch die enzymatische Wirkung des Sauerstoffs bei Kontakt mit dem Extraktionsgut 42 eine negative Wirkung auf die Wirkstoffe haben würde.

In allen Beispielen der Fig. 1 - 3 wird als Lösungsmittel 16 etwa 94%iges Ethanol und Wasser gemischt, die Zusammensetzung variiert mit dem Wassergehalt der Pflanzen 40, damit das Endextrakt 16a einen definierten Alkoholgehalt aufweist.

Sowohl der Rührkessel 12 als auch die Schnecke 64 können mit verschiedenen Zerkleinerern 36 kombiniert werden, z.B. einem diskontinuierlich arbeitenden Turboschneider oder einem kontinuierlich arbeitenden Walzenkneter. Die Pflanzen oder Pflanzenteile 40 werden jeweils unter Niveau, also mit wenig Lösungsmittel, auf vorzugsweise etwa 0,5 - 5 mm mit grosser Grenzfläche, zerkleinert und ebenfalls unter Niveau in einen Extraktor geführt.

Vergleiche mit bisher üblichen Extraktionsverfahren, insbesondere dem Mazerationsverfahren, bei welchem Pflanzen oder Pflanzenteile 40 bei Zimmertemperatur in ein ruhendes Lösungsmittel eingetaucht werden, zeigen fast durchwegs bessere bis deutlich bessere Resultate für die vorliegende Erfindung. Bei einer Schnecken-Extraktoranlage 60 mit kontinuierlicher Förderung des feinstückigen Pflanzenmaterials 42 gelegentlich auftretende Verstopfungen können durch Optimalisierung der Zufuhrrate, der Drehzahl der Schnecke 64 und des Beladungsgrads beseitigt werden. Die Auswertung der Ausführungsbeispiele hat folgende grundsätzlichen Resultate ergeben:
- Der Trockenmasse- und Leitwirkstoffgehalt des Extraktes 16a kann in der Regel durch einen Verlängerung der Extraktionszeit erhöht werden.
- Die enzymatische Zersetzung ist beim Rührkessel 12 wegen der grösseren Turbulenzen grösser als beim Schnecken-Extraktor 62.
- Höhere Drehzahlen des Rührers 44 bewirken einen erhöhten Abbau der empfindlichen enzymatischen Wirkstoffe durch den gelösten Sauerstoff, auch wenn die ganze Anlage zur Eliminierung des Luftsauerstoffs mit Inertgas begast wird.
- Eine kontinuierliche, unmittelbar in den Extraktor führende Zerkleinerung ist optimal, in der Regel erbringt ein Turboschneider die besseren Resultate als andere Zerkleinerungsmaschinen.
- Bei einem Rührkessel 12 erhöht eine Fraktionierung des Lösungsmittels 16 die Extraktionsqualität.
- In einem Schneckenextraktor 62 wird die Extraktion bei einer tieferen Drehzahl der Schnecke 64 und/oder bei deren Verlängerung verbessert.
- In einem Rührkessel wird die Qualität der Extraktion deutlich verbessert, wenn das Lösungsmittel 16 rezykliert wird und eine höhere Beladung mit Wirkstoffen hat.
- Der Einfluss der Zerkleinerungmethode nimmt bei längerer Extraktionszeit ab, weil das feinstückige Pflanzenmaterial 42a während der Extraktion weiter zerkleinert wird.

Erfindungsgemäss wurden bisher sehr gute Resultate mit einer Doppelschnecken-Extraktoranlage 60 mit den folgenden Daten erzielt:
- Schneckenlänge 3 - 3,5 m
- Schneckendurchmesser etwa 150 mm
- Steigung der Schnecke 30 - 50 mm
- Drehzahl der Schnecke 0,5 - 1 pro Minute
- Beladungsgrad etwa 70 %
- Extraktionszeit etwa 20 Minuten

Mit diesem Doppelmischschneckenextraktor treten auch keine Verstopfungsprobleme mit dem feinstückigen Pflanzenmaterial 42 im Extraktor auf.

## Patentansprüche

1. Verfahren zur Fest-Flüssig-Extraktion von Wirkstoffen aus fettarmen Pflanzen oder Pflanzenteilen (40) mit einem Extraktionslösungsmittel (16) zur Herstellung von Pflanzenextrakten (16a),
**dadurch gekennzeichnet, dass**
frischgeschnittene, fettarme Pflanzen oder Pflanzenteile (40) unter Beibehaltung von deren Grundstruktur zu einem heterogenen Flüssig-Fest-Suspensionssystem mit feinstückigem Pflanzenmaterial (42) zerkleinert und die Wirkstoffe im gleichen Arbeitsgang kontinuierlich oder sequentiell mit wenigstens einem Lösungsmittel (16) bei Zimmertemperatur extrahiert und angereichert werden.

2. Verfahren nach Anspruch 1, **dadurch gekennzeichnet, dass** schon vor dem und/oder beim Zerkleinerungsvorgang etwas Lösungsmittel zugegeben, also unter Niveau geschnitten wird.

3. Verfahren nach Anspruch 1 oder 2, **dadurch gekennzeichnet, dass** die Pflanzen oder Pflanzenteile (40) schnell und kräftig auf eine möglichst homogene Stückgrösse geschnitten werden.

4. Verfahren nach einem der Ansprüche 1 bis 3, **dadurch gekennzeichnet, dass** die Pflanzen und Pflanzenteile (40) bis zum Erreichen einer pflanzenspezifischen Stückgrösse von 0,1 - 20 mm, vorzugsweise von 0,5 - 5 mm, insbesondere von etwa 3 mm, geschnitten werden.

5. Verfahren nach einem der Ansprüche 1 bis 4, **dadurch gekennzeichnet, dass** bei kontinuierlicher Extraktion das laufend transportierte feinstückige Pflanzenmaterial (42,42a) vom im Endprodukt verbleibenden Lösungsmittel (16) im Gegen- oder Kreuzstrom umflossen wird.

6. Verfahren nach einem der Ansprüche 1 bis 5, **dadurch gekennzeichnet, dass** die kontinuierliche Extraktion von laufend gefördertem, feinstückigem Pflanzenmaterial (42,42a) in einem Schneckenextraktor (62), vorzugsweise in einem Doppelschneckenextraktor, erfolgt.

7. Verfahren nach einem der Ansprüche 1 bis 4, **dadurch gekennzeichnet, dass** die vorzugsweise sequentielle Extraktion von stationärem, feinstückigem Pflanzenmaterial (42) in einer Rührkessel-Extraktionsanlage (10), insbesondere in einer Rührkesselkaskade (12), erfolgt, welche von mehrfach rezykliertem Extrakt (16a) durchflossen wird.

8. Verfahren nach einem der Ansprüche 1 bis 7, **dadurch gekennzeichnet, dass** die Extraktion in einer Inertgasatmosphäre (34), insbesondere aus Argon oder Stickstoff, durchgeführt wird.

9. Verfahren nach einem der Ansprüche 1 bis 8, **dadurch gekennzeichnet, dass** die Extraktion mit einem anorganischen und/oder organischen flüssigen Lösungsmittel (16), vorzugsweise Wasser, Hexan und/oder wenigstens einem Alkohol durchgeführt wird, insbesondere Glyzerin, einer Mischung Ethanol/Wasser oder einer Emulsion Hexan/Wasser.

10. Verfahren nach einem der Ansprüche 1 bis 9, **dadurch gekennzeichnet, dass** die mittlere Extraktionszeit des feinstückigen Pflanzenmaterials (42) im Extraktor (12,62) unterhalb der enzymatischen Zersetzungszeit der Wirkstoffe liegt, vorzugsweise im Bereich von 10 - 60 Minuten, insbesondere von 20 - 30 Minuten.

11. Verfahren nach einem der Ansprüche 1 bis 10, **dadurch gekennzeichnet, dass** die Parameter, insbesondere die Extraktionszeit, Temperatur und Schubspannungen, anhand von standardmässigen quantitativen oder halbquantitativen analytischen Techniken, vorzugsweise Dünnschichtchromatografie-Analysen, laufend optimiert werden.

12. Verfahren nach einem der Ansprüche 1 bis 11, **dadurch gekennzeichnet, dass** der Trester (78), die Pflanzenreste ohne die extrahierten Wirkstoffe, ausgepresst wird.

13. Verwendung von mit dem Verfahren nach einem der Ansprüche 1 bis 12 hergestellten flüssigen Pflanzenextrakten (16a) zur Herstellung von wenigstens teilweise natürlichen Arzneimitteln, Lebensmitteln oder Kosmetika.

## Claims

1. A process for the solid-liquid extraction of active substances from low-fat plants or plant parts (40) with an extraction solvent (16) for the production of plant extracts (16a), **characterized in that** freshly harvested, low-fat plants or plant parts (40) are chopped up into a heterogeneous liquid-solid suspension system containing finely cut up plant material (42), while retaining their basic cell structure, and the active substances in a single operation are continuously or sequentially extracted and enriched into at least one solvent (16) at room temperature.

2. A process according to claim 1, **characterized in that that** already before and/or during the chopping operation some solvent is added, so that the chopping takes place in the solvent.

3. A process according to claim 1 or 2, **characterized in that** the plants or the plant parts (40) are rapidly and vigorously cut up to a particle size that is as homogeneous as possible.

4. A process according to any of claims 1 to 3, **characterized in that** the plants or plant parts (40) are chopped up to a plant-specific particle size of 0.1 - 20 mm, preferably 0.5 -5 mm, in particular 3 mm.

5. A process according to any of claims 1 to 4, **characterized in that** during a continuous extraction the continuously transported finely cut up plant material (42, 42a) is bathed in a counter- or crossflowing solvent, that remains present in the final product.

6. A process according to any of claims 1 to 5, **characterized in that** the continuous extraction of continuously transported finely cut up plant material (42, 42a) takes place in a screw extractor (62), preferably in a twin-screw extractor.

7. A process according to any of claims 1 to 4, **characterized in that** the preferably sequential extraction of stationary finely cut up plant material (42) takes place in a stirred- tank extraction device (10), in particular in a cascade of tank reactors (12) in which the multiply recycled extract (16a) circulates.

8. A process according to any of claims 1 to 7, **characterized in that** the extraction is carried out in an inert gas atmosphere, in particular a nitrogen or argon atmosphere.

9. A process according to any of claims 1 to 8, **characterized in that** the extraction is carried out with an inorganic and/or organic liquid solvent (16), preferably water, hexane and/or at least one alcohol, in particular glycerine, a mixture of ethanol/water or an emulsion of hexane/water.

10. A process according to any of claims 1 to 9, **characterized in that** the average duration of the extraction of finely cut up plant material (42) in the extractor (12, 62) is shorter than the duration of the enzymatic degradation of the active substances, preferably between 10 and 60 minutes, in particular between 20 and 30 minutes.

11. A process according to any of claims 1 to 10, **characterized in that** the relevant parameters, in particular the extraction duration, temperature and shear stresses, are continuously optimized with the help of standard quantitative or semi-quantitative analytical techniques, in particular thin layer chromatographic analysis.

12. A process according to any of claims 1 to 11, **characterized in that** the plant residue (78) from which the active substances have been extracted, is pressed.

13. The use of the liquid plant extracts (16a) prepared according to any of claims 1 to 12 for the production of at least partially natural drugs, foodstuffs or cosmetics.

## Revendications

1. Procédé pour l'extraction solide-liquide de principes actifs de plantes ou de parties de plantes pauvres en matières grasses (40) avec un solvant d'extraction (16) pour la fabrication d'extraits végétaux (16a),
**caractérisé en ce que**
des plantes ou parties de plantes pauvres en matières grasses, fraîchement découpées sont réduites en un système de suspension liquide-solide hétérogène contenant de la matière végétale finement découpée (42), en maintenant leur structure de base , et que les principes actifs sont extraits et enrichis lors de la même étape du procédé de manière continue ou séquentielle avec au moins un solvant (16) ci température ambiante.

2. Procédé selon la revendication 1,
**caractérisé en ce que**
déjà avant et/ou lors du processus de comminution peu de solvant est ajouté, de telle sorte que la découpe a lieu sous niveau.

3. Procédé selon la revendication 1 ou 2,
**caractérisé en ce que**
les plantes ou les parties de plantes (40) sont découpées rapidement et vigoureusement pour arriver à une taille des particules aussi homogène que possible.

4. Procédé selon l'une des revendications 1 à 3,
**caractérisé en ce que**
les plantes et parties de plantes (40) sont découpées jusqu'à ce qu'une taille de particules spécifique à la plante de 0,1 - 20 mm, de préférence de 0,5 - 5 mm, en particulier environ 3 mm, soit atteinte.

5. Procédé selon l'une des revendications 1 à 4,
**caractérisé en ce que**
lors d'une extraction en continu la matière végétale finement découpée (42, 42a), laquelle est transportée en continu, baigne dans le solvant circulant à contre-courant ou à courant croisé et restant dans le produit final (16).

6. Procédé selon l'une des revendications 1 à 5,
**caractérisé en ce que**
l'extraction en continu de matière végétale finement découpée (42, 42a), laquelle est transportée en continu, a lieu dans un extracteur à vis (62), de préférence un extracteur à double vis.

7. Procédé selon l'une des revendications 1 à 4,
**caractérisé en ce que**
l'extraction de préférence séquentielle de matière végétale finement découpée stationnaire (42) a lieu dans une installation d'extraction à cuve agitée (10), en particulier dans une cascade de cuves agitées (12), dans laquelle circule un extrait plusieurs fois recyclé (16a).

8. Procédé selon l'une des revendications 1 à 7,
**caractérisé en ce que**
l'extraction a lieu sous atmosphère inerte (34), en particulier une atmosphère d'argon ou d'azote.

9. Procédé selon l'une des revendications 1 à 8,
**caractérisé en ce que**
l'extraction est effectuée avec un solvant liquide inorganique et/ou organique, de préférence eau, hexane et/ou au moins un alcool, en particulier glycérine, un mélange éthanol/eau ou une émulsion hexane/eau.

10. Procédé selon l'une des revendications 1 à 9,
**caractérisé en ce que**
la durée d'extraction moyenne de la matière végétale finement découpée (42) dans l'extracteur (12, 82), est inférieure à la durée de la dégradation enzymatique des principes actifs, et se trouve de préférence dans le domaine 10 - 60 minutes, en particulier 20 - 30 minutes.

11. Procédé selon l'une des revendications 1 à 10,
**caractérisé en ce que**
les paramètres, en particulier la durée d'extraction, la température et les tensions de poussée, sont optimisés en permanence à l'aide de techniques analytiques quantitatives ou semi-quantitatives standards, de préférence d'analyses par chromatographie par couches minces.

12. Procédé selon l'une des revendications 1 à 11,
**caractérisé en ce que**
le résidu (78), les restes de plantes épuisées en principes actifs, est pressé.

13. Utilisation des extraits végétaux liquides (16a) obtenus par le procédé selon l'une des revendications 1 à 12 pour la fabrication de produits pharmaceutiques, alimentaires ou cosmétiques au moins partiellement naturels.
